# EUROPEAN PATENT SPECIFICATION

(11) **EP 3 221 305 B1**
(45) Date of publication and mention of the grant of the patent: **07.03.2018**
(21) Application number: 16708416.9
(22) Date of filing: 08.03.2016
(51) Int. Cl.: C07D 401/12, C07D 257/04

(54) **METHOD FOR PREPARATION OF CERTAIN 1,5 DISUBSTITUTED TETRAZOLES**
VERFAHREN ZUR HERSTELLUNG BESTIMMTER 1,5-DISUBSTITUIERTER TETRAZOLE
PROCÉDÉ DE PRÉPARATION DE CERTAINS TÉTRAZOLES 1,5 DISUBSTITUÉS

(30) Priority: 10.03.2015 US 201562130877 P; 10.03.2015 EP 15158452; 11.03.2015 EP 15158620; 30.06.2015 EP 15174511; 17.07.2015 EP 15177356; 10.08.2015 EP 15180374
(43) Date of publication of application: 27.09.2017
(73) Proprietor: Lonza Ltd, 3930 Visp (CH)
(72) Inventor: ZARAGOZA DOERWALD, Florencio, 3930 Visp (CH)
(86) International application number: PCT/EP2016/054861
(87) International publication number: WO 2016/142364

(56) References cited:
- EP-A1- 2 407 461
- WO-A1-2011/110651
- G ADIWIDJAJA ET AL: "a-Oxo-und a-Thioxothioamide aus Methylketonen", LIEBIGS ANNALEN DER CHEMIE, 1 January 1983 (1983-01-01), pages 1116-1132, XP055198621, cited in the application

## Description

The invention discloses a method for the preparation of certain 1,5 disubstituted tetrazoles and similar compounds starting from certain thioacetamides, which ultimately allows the preparation of these certain 1,5 disubstituted tetrazoles from readily available compounds such as acetophenone and similar compounds.

### BACKGROUND OF THE INVENTION

Picarbutrazox with CAS 500207-04-5 is a fungicide useful as plant protecting agent. A key intermediate for the preparation of picarbutrazox is 1-methyl-5-benzoyltetrazole. EP 2546236 A1 discloses the steps from 1-methyl-5-benzoyltetrazole to picarbutrazox.

EP 2407461 A1 discloses a method for preparation of 1-methyl-5-benzoyltetrazole from methyl 2-oxo-2-phenylacetate, via intermediate conversion of N-methyl-2-oxo-2-phenylacetamide into an imidoyl chloride. Methyl 2-oxo-2-phenylacetate, however, is an expensive intermediate. Moreover, amides are unreactive compounds, and the conversion of amides into imidoyl chlorides usually requires elevated reaction temperatures, causing the formation of byproducts and low yields of the required imidoyl chloride. The reaction times disclosed in EP 2407461 A1 are rather long. The reaction temperatures are rather high

WO 2011/110651 A1 discloses a method for preparation of 1-methyl-5-benzoyltetrazole starting from benzoylchloride. The method uses methyl isocyanide. Methyl isocyanide is a substance that is problematic to use in large scale production due its high toxicity, high volatility, and high reactivity. The production of methyl isocyanide usually causes the formation of large amounts of phosphorus-containing waste, which is expensive to dispose of.

G. Adiwidjaja et al., Liebigs Ann. Chem. 1983, 1116-1132, discloses a method for the preparation of alpha-ketothioamides by reaction of a ketone with thionyl chloride and further addition of an amine.

There was a need for a method that does not use methyl isocyanide or methyl 2-oxo-2-phenylacetate.

Unexpectedly a method for preparation of certain 1,5 disubstituted tetrazols was found starting from certain thioacetamides, which ultimately allows the preparation of these certain 1,5 disubstituted tetrazoles also from readily available compounds such as acetophenone and similar compounds.

The method does not use methyl isocyanide or methyl 2-oxo-2-phenylacetate.

It is not a common strategy for the enhancement of the reactivity of amides to convert amides into thioamides. Thioamides have a thoroughly different reactivity than amides. E.g. thioamides are readily alkylated at the sulfur atom, and are readily oxidized to a variety of different products. R. N. Hurd et al., Chem. Rev. 1961, 61, 45-86, discloses a variety of such reactions of thioamides which are not available for the corresponding amides. Specifically on page 78 under paragraph 4 it is disclosed that e.g. thionbenzamide dimerizes upon treatment with thionyl chloride, in case of thionbenzanilide two molecules are connected by a oxidative dimerization. Thionacetamide reacts again in a different way: no condensation products were found; only decomposition products such as acetamide, sulfur, sulfur dioxide, and hydrogen chloride were isolated.
Furthermore no examples of a conversion of ketothioamides into acyltetrazoles have been reported in the literature. Therefore it was not predictable or expected from prior art that a ketothioamide could be converted to the target tetrazole.

Furthermore when thioamides are prepared from amides, e.g. with P₄S₁₀, this conversion involves an additional synthetic step, additional costs and creates additional waste. Therefore such an extra step would increase the overall costs of a synthesis significantly. In addition a conversion of amides into thioamides. e.g. with P₄S₁₀, is not compatible with other carbonyl groups. Therefore the ketoamide methyl 2-oxo-2-phenylacetate Ph-CO-C(O)-N(H)-Me that is used in EP 2407461 A1 for the preparation of the target tetrazole cannot be readily converted into the respective ketothioamide Ph-CO-C(S)-N(H)-Me. For the skilled person the ketothioamide Ph-CO-C(S)-N(H)-Me is for this reason not an obvious activated form of the ketoamide Ph-CO-C(O)-N(H)-Me, which would automatically be considered to be synthetic equivalents, and the expert would therefore not have obviously or automatically identified the ketothioamide Ph-CO-C(S)-N(H)-Me as an obvious activated form of the ketoamide Ph-CO-C(O)-N(H)-Me.

These facts show that the skilled person in instant case would not expect or assume that the ketothioamide would have very similar chemical properties as the ketoamide, or that the two compounds could be interchanged without influencing the remaining features of the compounds, especially not without influencing the process. In fact, the skilled person knows that replacing an oxygen atom by a sulfur atom in a carbonyl group is not a generally or a commonly used alternative and cannot be automatically expected to yield the same results or would allow to use the same process.

It required inventive skill to discern that (1) the ketothioamide Ph-CO-C(S)-N(H)-Me could be used instead of the ketoamide Ph-CO-C(O)-N(H)-Me, that is that the ketothioamide actually would react in a similar way as the ketoamide with thionylchloride, and that (2) the ketothioamide would represent a cost effective alternative, even though it was not availabale from the ketoamide, since it was available from acetophenone and didn't need to be prepared from the ketoamide by an exchange of O against S. In addition as a point (3) the skilled person needed to see that there was a problem at all, that is that there was a potential for improvement at all, and that there was a less expensive alternative to methyl 2-oxo-2-phenylacetate, and that there was a potential to arrive at a method which can be done at lower temperatures and with shorter reaction times.

In the following text,
ambient pressure means usually 1 bar, depending on the weather;
halide means F⁻ , Cl⁻ , Br⁻ or I⁻, preferably Cl⁻ , Br⁻ or I⁻, more preferably Cl⁻ or Br⁻; halogen means F, Cl, Br or I, preferably Cl, Br or I;
if not otherwise stated.

### SUMMARY OF THE INVENTION

Subject of the invention is a method for the preparation of compound of formula (IV); 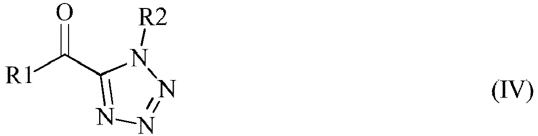 the method comprises four steps, a step ST1, a step ST2, a step ST3 and a step ST4;
ST4 comprises a reaction REAC4 of a compound of formula (III) with a compound AZID; 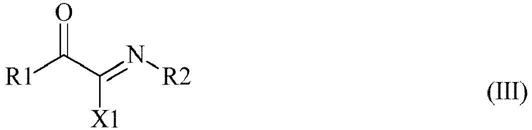
AZID is selected from the group consisting of alkali metal azide, alkali earth metal azide, [N(R10)(R11)(R12)R13]⁺ [N₃]⁻ and guanidinium azide;
R10, R11, R12 and R13 are identical or different and independently from each other selected from the group consisting of H and C₁₋₈ alkyl;
XI is selected from the group consisting of Cl, Br, S-CH₃, S-CH₂-CH₃, S-CH₂-Phenyl, S-C(O)-C(CH₃)₃, and SO₃H;
REAC4 results in compound of formula (IV);
compound of formula (III) is prepared in ST3;
ST3 comprises a reaction REAC3 of a compound of formula (II) with a compound COMPREAC3; 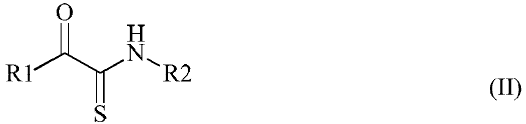 COMPREAC3 is selected from the group consisting of thionyl chloride, COCl₂, diphosgene, triphosgene, POCl₃, PCl₃, PCl₅, POBr₃, PBr₃, PBr₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, alkali metal persulfate salt, Cl₂, Br₂, and I₂; REAC3 results in compound of formula (III);
wherein
R1 is selected from the group consisting of
   phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
   5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy;
R2 is selected from the group consisting of
   C₁₋₁₂ alkyl, the C₁₋₁₂ alkyl being unsubstituted or substituted by 1, 2 or 3 substituents selected from the group consisting of phenyl or 5 or 6 membered aromatic heterocycle with 1, 2 or 3 heteroatoms selected from the group consisting of N, O and S,
   C₃₋₆ cycloalkyl,
   phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
   5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy;
compound of formula (II) is prepared in ST2;
ST1 comprises a reaction REAC1 of compound of formula (I) with SOCl₂; 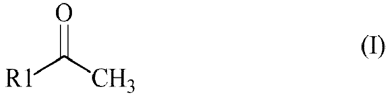 REAC1 results in a reaction product REACPROD1;
ST2 comprises a reaction REAC2 of REACPROD1 with R2-NH₂;
REAC2 results in compound of formula (II).

### DETAILED DESCRIPTION OF THE INVENTION

Preferably, R1 is selected from the group consisting of
phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₆ alkyl, and C₁₋₆ alkoxy;
more preferably, R1 is selected from the group consisting of
phenyl, the phenyl being unsubstituted or substituted by 1, 2 or 3 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, trifluoromethyl, C₁₋₆ alkyl, and methoxy;
even more preferably, R1 is selected from the group consisting of
phenyl, the phenyl being unsubstituted or substituted by 1 or 2 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N and S, the 5 or 6 membered aromatic heterocycle being unsubstituted or substituted by 1 or 2 substituents selected from the group consisting of halogen, nitro, trifluoromethyl, C₁₋₆ alkyl and methoxy.

Preferably, R2 is selected from the group consisting of
C₁₋₆ alkyl, the C₁₋₆ alkyl being unsubstituted or substituted by 1 substituent selected from the group consisting of phenyl or 5 or 6 membered aromatic heterocycle with 1, 2 or 3 heteroatoms selected from the group consisting of N, O and S,
C₃₋₆ cycloalkyl,
phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₆ alkyl, and C₁₋₆ alkoxy;
more preferably, R2 is selected from the group consisting of
methyl, ethyl, n-propyl, iso-propyl, butyl and pentyl, the methyl, ethyl, n-propyl, iso-propyl, butyl and pentyl being unsubstituted or substituted by 1 substituent selected from the group consisting of phenyl or 5 or 6 membered aromatic heterocycle with 1, 2 or 3 heteroatoms selected from the group consisting of N, O and S,
C₅₋₆ cycloalkyl,
phenyl, the phenyl being unsubstituted or substituted by 1, 2 or 3 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, trifluoromethyl, C₁₋₆ alkyl, and methoxy;
even more preferably, R2 is selected from the group consisting of
methyl, ethyl, n-propyl and iso-propyl, the methyl, ethyl, n-propyl and iso-propyl being unsubstituted or substituted by 1 substituent selected from the group consisting of phenyl or 5 or 6 membered aromatic heterocycle with 1, 2 or 3 heteroatoms selected from the group consisting of N, O and S,
C₅₋₆ cycloalkyl,
phenyl, the phenyl being unsubstituted or substituted by 1 or 2 substituents selected from the group consisting of halogen, nitro, trifluoromethyl, C₁₋₆ alkyl, and methoxy, and
5 or 6 membered aromatic heterocycle with 1, 2 or 3 heteroatoms selected from the group consisting of N, O and S, the 5 or 6 membered aromatic heterocycle being unsubstituted or substituted by 1 or 2 substituents selected from the group consisting of halogen, nitro, trifluoromethyl, C₁₋₆ alkyl, and methoxy;
Preferably, AZID is selected from the group consisting of alkali metal azide, and [N(R10)(R11)(R12)R13]⁺ [N₃]⁻.
More preferably, AZID is selected from the group consisting of alkali metal azide, and [N(R10)(R11)(R12)R13]⁺ [N₃]⁻; and
R10 is C₁₋₄ alkyl; and
R11, R12 and R13 are identical or different and independently from each other selected from the group consisting of H and C₁₋₄ alkyl.
Even more preferably, AZID is selected from the group consisting of sodium azide, tetrabutylammonium azide, and triethylammonium azide.

Preferably, the molar amount of AZID is from 1 to 20 times, more preferably from 1 to 15 times, and even more preferably from 1 to 10 times, based on the molar amount of compound of formula (III).

Preferably, REAC4 is done at a temperature of from -60 °C to 100 °C, more preferably from -30 °C to 60 °C, even more preferably from from -10 °C to 40 °C.
Preferably, REAC4 is done at a pressure of from ambient pressure to 10 bar, more preferably from ambient pressure to 5 bar, even more preferably from ambient pressure to 2 bar.
Preferably, the reaction time of REAC4 is from 5 min to 24 h, more preferably from 15 min to 12 h, even more preferably from 30 min to 8 h.

Preferably, REAC4 is done in a solvent SOLV4; SOLV4 is selected from the group consisting of water, acetone, acetonitrile, ethanol, methanol, ethylene glycol, benzene, toluene, chlorobenzene, N,N-dimethylformamide, NMP, THF, dioxane, ethyl acetate, butyl acetate, and mixtures thereof;
more preferably, SOLV4 is selected from the group consisting of water, acetone, ethanol, methanol, benzene, toluene, chlorobenzene, THF, dioxane, ethyl acetate, butyl acetate, and mixtures thereof;
even more preferably, SOLV4 is selected from the group consisting of water, benzene, toluene, chlorobenzene, ethyl acetate, butyl acetate, and mixtures thereof.

Preferably, the weight of SOLV4 is from 0.01 to 50 times, more preferably from 0.05 to 30 times, even more preferably from 0.1 to 20 times, of the weight of compound of formula (III).

REAC4 can be done in the presence of a compound PTCS4, PTCS4 is a compound of formula [PTCS4];

[N(R20)(R21)(R22)R23]⁺ [X2]⁻ [PTCS4]

R20, R21, R22 and R23 are identical or different and independently from each other selected from the group consisting of H, C₁₋₂₀ alkyl, phenyl and benzyl;
[X2] is selected from the group consisting of halide and hydrogensulfate.
More preferably, PTCS4 is selected from the group consisting of tetrabutylammonium halide, tetrabutylammonium hydrogensulfate, cetyltrimethylammonium chloride and bromide, and benzyltributylammonium chloride and bromide;
even more preferably, PTCS4 is selected from the group consisting of tetrabutylammonium chloride and bromide, tetrabutylammonium hydrogensulfate, cetyltrimethylammonium chloride, and benzyltributylammonium chloride.

Preferably, the molar amount of PTCS4 is from 0.001 to 1.0 times, more preferably from 0.005 to 0.8 times, and even more preferably from 0.01 to 0.5 times, based on the molar amount of compound of formula (III).

In one possible embodiment, REAC4 is done by mixing compound of formula (III) dissolved in SOLV4, SOLV4 except water, with AZID dissolved in water, thereby the reaction mixture in REAC4 has two liquid phases. In this embodiment PTCS4 can be used.
In another embodiment REAC4 is done by mixing compound of formula (III) dissolved in SOLV4, SOLV4 except water, with AZID in solid form. Also in this embodiment PTCS4 can be used.

The method of instant invention allows the use of PTCS4, but also works without the use of PTCS4.
Therefore in one embodiment, no PTCS4 is used in REAC4.
In another embodiment, no Bu₃N-CH₂-Ph or a chloride thereof is used.
In another embodiment, no PTCS4 and no Bu₃N-CH₂-Ph and no chloride of Bu₃N-CH₂-Ph is used.
In another embodiment, no phase transfer catalyst is used in REAC4.

When REAC4 is conducted in the presence of an aqueous phase, the pH of said aqueous phase can be adjusted by addition of a buffer. Preferably, the pH of the aqueous phase of REAC4 is between 0 and 14, more preferably between 2 and 10, and even more preferably between 3 and 8.
Preferably, when a buffer is used in REAC4, a buffer BUFF4 is used, BUFF4 is selected from the group consisting of acetate buffer, phosphate buffer, carbonate buffer, sulfate buffer, and mixtures thereof;
more preferably, BUFF4 is selected from the group consisting of acetate buffer, phosphate buffer and sulfate buffer.

Preferably, the molar amount of BUFF4 is from 0.01 to 20 times, more preferably from 0.05 to 6 times, and even more preferably from 0.1 to 3 times, based on the molar amount of compound of formula (III).

Compound of formula (IV) can be isolated after REAC4 by any conventional method, for instance by extraction and by crystallization. Preferably, any volatile byproduct is distilled off, and the residue is purified or used without further purification.

Preferably, COMPREAC3 is selected from the group consisting of thionyl chloride, COCl₂, diphosgene, triphosgene, POCl₃, PCl₃, PCl₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, sodium persulfate salt, potassium persulfate salt, Cl₂, Br₂, and I₂;
more preferably, COMPREAC3 is selected from the group consisting of thionyl chloride, COCl₂, triphosgene, POCl₃, PCl₃, PCl₅, S₂Cl₂, methyl chloride, methyl bromide, methyl iodide, dimethyl sulfate, trimethylphosphate, methyl methanesulfonate, methyl benzenesulfonate, methyl tosylate, hydrogen peroxide, C₁₋₄ alkyl hydro peroxide, C₁₋₆ alkaloyl peroxide, perbenzoic acid, 3-chloro-perbenzoic acid, potassium persulfate salt, Cl₂, Br₂, and I₂;
even more preferably, COMPREAC3 is selected from the group consisting of thionyl chloride, COCl₂, triphosgene, POCl₃, S₂Cl₂, methyl chloride, dimethyl sulfate, trimethylphosphate, methyl benzenesulfonate, methyl tosylate, hydrogen peroxide, tert-butylhydroperoxide, peracetic acid, 3-chloro-perbenzoic acid, potassium persulfate salt, Cl₂, and Br₂;
especially, COMPREAC3 is thionyl chloride.

Preferably, XI is selected from the group consisting of Cl, S-CH₃, S-CH₂-CH₃, S-CH₂-Phenyl, S-C(O)-C(CH₃)₃, and SO₃H;
more preferably, XI is selected from the group consisting of Cl, S-CH₃, and SO₃H;
even more preferably, XI is Cl.

Preferably, the molar amount of COMPREAC3 is from 1 to 50 times, more preferably from 1 to 30 times, and even more preferably from 1 to 20 times, based on the molar amount of compound of formula (II).

Preferably, REAC3 is done at a temperature of from -30 °C to 120 °C, more preferably from -10 °C to 90 °C, even more preferably from 0 °C to 60 °C, especially from 0 °C to 50 °C, more especially from 0 °C to 40 °C, even more especially from 0 °C to 30 °C.
Preferably, REAC3 is done at a pressure of from ambient pressure to 10 bar, more preferably from ambient pressure to 5 bar, even more preferably from ambient pressure to 2 bar.
Preferably, the reaction time of REAC3 is from 5 min to 24 h, more preferably from 10 min to 12 h, even more preferably from 30 min to 10 h, especially from 30 min to 8 h, more especially from 30 min to 7 h, even more especially from 30 min to 6 h, in particular from 30 min to 5 h, more in particular from 30 min to 4 h, even more in particular from 30 min to 3 h.

REAC3 can be done neat or in a solvent.
If REAC3 is done in a solvent, it is done preferably in is done in a solvent SOLV3, SOLV3 is selected from the group consisting of water, pyridine, 3-picoline, acetone, acetonitrile, benzene, toluene, chlorobenzene, N,N-dimethylformamide, NMP, THF, dioxane, ethyl acetate, butyl acetate, and mixtures thereof;
more preferably, SOLV3 is selected from the group consisting of water, pyridine, 3-picoline, acetone, benzene, toluene, chlorobenzene, THF, dioxane, ethyl acetate, butyl acetate, and mixtures thereof;
even more preferably, SOLV3 is selected from the group consisting of water, pyridine, 3-picoline, benzene, toluene, chlorobenzene, THF, dioxane, and mixtures thereof.

Preferably, the weight of SOLV3 is from 0.01 to 50 times, more preferably from 0.05 to 30 times, even more preferably from 0.1 to 20 times, of the weight of compound of formula (II).

In a especial embodiment,
COMPREAC3 is thionyl chloride and
XI is Cl.

Especially,
R1 is phenyl; and
R2 is methyl;
more especially,
COMPREAC3 is selected from the group consisting of thionyl chloride, COCl₂, hydrogen peroxide, S₂Cl₂, and Cl₂;
XI is Cl or SO₃H;
AZID is sodium azide;
R1 is phenyl; and
R2 is methyl;
even more especially,
COMPREAC3 is thionyl chloride;
XI is Cl;
AZID is sodium azide;
R1 is phenyl;
R2 is methyl.

Compound of formula (III) can be isolated after REAC3 by any conventional method, for instance by extraction, distillation under reduced pressure, or by crystallization. Preferably, any volatile byproduct is distilled off, and the residue is used without further purification.

Preferably, the molar amount of R2-NH₂ in REAC2 is from 1 to 20 times, more preferably from 1 to 15 times, and even more preferably from 1 to 12 times, based on the molar amount of compound of formula (I).

Preferably, REAC2 is done at a temperature of from -40 °C to 100 °C, more preferably from -20 °C to 80 °C, even more preferably from from -10 °C to 50 °C.
Preferably, REAC2 is done at a pressure of from ambient pressure to 10 bar, more preferably from ambient pressure to 5 bar, even more preferably from ambient pressure to 2 bar.
Preferably, the reaction time of REAC2 is from 5 min to 48 h, more preferably from 20 min to 24 h, even more preferably from 30 min to 18 h, especially from 30 min to 10 h.

REAC2 can be done neat or in a solvent.
If REAC2 is done in a solvent, it is done preferably in a solvent SOLV2, SOLV2 is selected from the group consisting of benzene, toluene, xylene, chlorobenzene, pyridine, 3-picoline, 2-methyl-5-ethylpyridine, chloroform, dichloromethane, THF, water, and mixtures thereof;
more preferably, SOLV2 is selected from the group consisting of benzene, toluene, xylene, chlorobenzene, pyridine, 3-picoline, 2-methyl-5-ethylpyridine, dichloromethane, THF, water, and mixtures thereof.

Preferably, the weight of SOLV2 is from 0.01 to 50 times, more preferably from 0.05 to 20 times, even more preferably from 0.1 to 10 times, of the weight of compound of formula (I).

REAC2 can be done in the presence of a base. If REAC2 is done in the presence of a base, then the base is a compound BAS2, BAS2 is selected from the group consisting of sodium carbonate, sodium bicarbonate, potassium carbonate, sodium hydroxide, potassium hydroxide, calcium hydroxide, and mixtures therof;
more preferably, BAS2 is selected from the group consisting of sodium carbonate, potassium carbonate, sodium hydroxide, calcium hydroxide, and mixtures therof.

Preferably, the molar amount of BAS2 is from 1 to 10 times, more preferably from 1 to 5 times, and even more preferably from 1 to 3 times, based on the molar amount of compound of formula (I).

Compound of formula (II) can be isolated after REAC2 by any conventional method, for instance by extraction, by distillation under reduced pressure, or by crystallization. Preferably, any volatile byproduct is distilled off, and the residue is purified by crystallization, or used without further purification.

Preferably, the molar amount of SOCl₂ in REAC1 is from 2 to 40 times, more preferably from 2 to 20 times, and even more preferably from 2 to 15 times, based on the molar amount of compound of formula (I).

Preferably, REAC1 is done at a temperature of from -20 °C to 80 °C, more preferably from -10 °C to 60 °C, even more preferably from from 0 °C to 40 °C.
Preferably, REAC1 is done at a pressure of from ambient pressure to 10 bar, more preferably from ambient pressure to 5 bar, even more preferably from ambient pressure to 2 bar.
Preferably, the reaction time of REAC1 is from 10 min to 72 h, more preferably from 30 min to 48 h, even more preferably from 1 h to 24 h.

REAC1 can be done neat or in a solvent.
If REAC1 is done in a solvent, it is done preferably in a solvent SOLV1, SOLV1 is selected from the group consisting of benzene, toluene, xylene, chlorobenzene, nitrobenzene, anisole, dichlorobenzene, dichloroethane, trichloroethane, dichloromethane, chloroform, acetonitrile, and mixtures thereof;
more preferably, SOLV1 is selected from the group consisting of benzene, chlorobenzene, toluene, dichloromethane, chloroform, acetonitrile, and mixtures thereof;
even more preferably, SOLV1 is selected from the group consisting of benzene, chlorobenzene, toluene, dichloromethane, chloroform, and mixtures thereof.

Preferably, the weight of SOLV1 is from 0.01 to 20 times, more preferably from 0.05 to 10 times, even more preferably from 0.1 to 5 times, of the weight of compound of formula (I).

REAC1 can be done in the presence of a catalyst. If REAC1 is done in the presence of a catalyst, then the catalyst is preferably a compound CAT1 or a salt of CAT1,
CAT1 is selected from the group consisting of pyridine, picoline, ethylmethylpyridine, 4-(dimethylamino)pyridine, 1,4-diazabicyclo[2.2.2]octane, N(R10)(R11)R12, N-methylmorpholine, N-ethyldiisopropylamine, Phe-N(R20)R21, and mixtures thereof;
R10, R11 and R12 are identical or different and are independently from each other C₁₋₈ alkyl;
R20 and R21 are identical or different and are independently from each other C₁₋₈ alkyl; and the salt of CAT1 is selected from the group consisting of hydrochloride salt, hydrobromide salt, acetate salt and trifluoroacetate salt.

Preferably, R10, R11 and R12 are identical or different and are independently from each other C₁₋₄ alkyl;
R20 and R21 are identical or different and are independently from each other C₁₋₂ alkyl.

More preferably, CAT1 is selected from the group consisting of pyridine, 2-picoline, 3-picoline, 4-picoline, 5-ethyl-2-methylpyridine, 4-(dimethylamino)pyridine, 1,4-diazabicyclo[2.2.2]octane, triethylamine, tributylamine, N-methylmorpholine, N-ethyldiisopropylamine, N,N-dimethylaniline, and mixtures thereof;
even more preferably, CAT1 is selected from the group consisting of pyridine, 3-picoline, 5-ethyl-2-methylpyridine, 4-(dimethylamino)pyridine, 1,4-diazabicyclo[2.2.2]octane, triethylamine, tributylamine, N-methylmorpholine, N-ethyldiisopropylamine, N,N-dimethylaniline, and mixtures thereof;
especially, CAT1 is selected from the group consisting of pyridine, 3-picoline, 5-ethyl-2-methylpyridine, 4-(dimethylamino)pyridine, 1,4-diazabicyclo[2.2.2]octane, triethylamine, tributylamine, N,N-dimethylaniline, and mixtures thereof;
more especially, CAT1 is selected from the group consisting of pyridine, 3-picoline, 5-ethyl-2-methylpyridine, N,N-dimethylaniline, and mixtures thereof.

Preferably, the salt of CAT1 is selected from the group consisting of hydrochloride salt, acetate salt and trifluoroacetate salt;
more preferably, the salt of CAT1 is selected from the group consisting of hydrochloride salt, and acetate salt.

Preferably, the molar amount of CAT1 is from 0.0001 to 0.6 times, more preferably from 0.0001 to 0.4 times, and even more preferably from 0.0001 to 0.2 times, based on the molar amount of compound of formula (I).

REACPROD1 can be isolated after REAC1 by any conventional method, for instance by extraction, by distillation under reduced pressure, or by crystallization. Preferably, any volatile byproduct is distilled off, and the residue is used without further purification.

Preferred embodiments of compound of formula (I), compound of formula (II), compound of formula (III) and compound of formula (IV) are compound of formula (1-1), compound of formula (II-1), compound of formula (III-1) and compound of formula (IV-1).

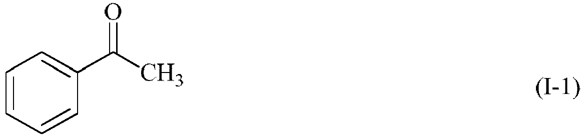

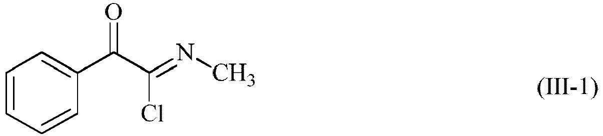

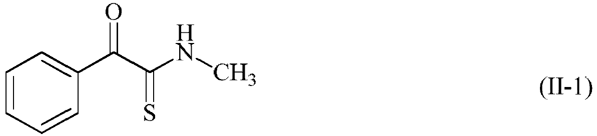

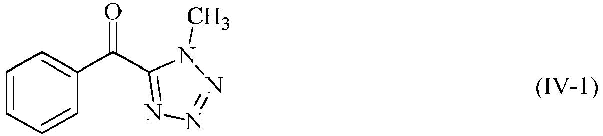

Further subject of the invention is a method for the preparation of compound of formula (VII), 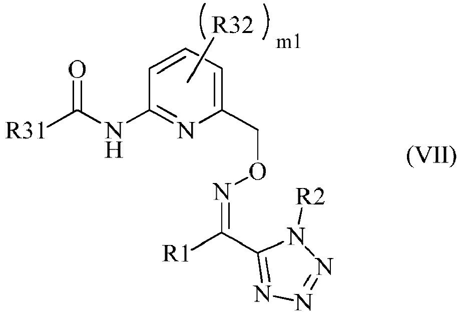 wherein the method comprises the two steps ST3 and ST4;
R31 is selected from the group consisting of linear, branched and cyclic C₁₋₁₀ alkyl and linear, branched and cyclic C₁₋₁₀ alkoxy, wherein the linear, branched and cyclic C₁₋₁₀ alkyl and the linear, branched and cyclic C₁₋₁₀ alkoxy can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH;
R32 is selected from the group consisting of F, Cl, Br, CN, NO₂, OH, SH, C(O)H, COOH, N(R50)R51,
   linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH,
   phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy,
   pyridinyl, pyridyl, pyrazolyl, imidazolyl, pyrrolidino, wherein the pyridinyl and the pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy,
   C₁₋₄ alkoxy, the C₁₋₄ alkoxy being unsubstituted or substituted by 1, 2 or 3 substituents selected from the group consisting of C₁₋₂ alkoxy, F and Cl,
   phenoxy,
   S(O)ₘ₂R41, C(O)R41 and CO₂R41;
   R41 is selected from the group consisting of
      N(R50)R51,
      linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH,
      phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy,
   pyridinyl, pyridyl, pyrazolyl, imidazolyl, pyrrolidino, wherein the pyridinyl and pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy,
   m2 is 0, 1 or 2;
   R50 and R51 are identical or different and selected from the group consisting of H, C₁₋₂ alkyl, C₁₋₄ alkoxycarbonyl and benzoyl;
m1 is 0, 1, 2 or 3;
   when m1 is 2 or 3, then the substituents R32 can be identical or different from each other; wherein ST3, ST4, R1 and R2 are as defined herein, also with all their embodiments;
further subject of the invention is a method for the preparation of compound of formula (VII),
   wherein the method comprises the two steps ST3 and ST4, and comprises the two steps ST1 and ST2;
   wherein STland ST2 are as defined herein, also with all their embodiments;
also with any individual embodiment or with any combination of two or more embodiments, the embodiments as described herein for any of these steps;
especially wherein
   - R1: is phenyl;
   - R2: is methyl;
   - R31: is tert-butoxy;
   - m1: is 0, that is no substituent R32.

Preferably, the preparation of compound of formula (VII) starting from compound of formula (IV) has three steps, a step ST5, a step ST6 and a step ST7, details for these three steps are disclosed in EP 2546236 A1.

ST5 comprises a reaction REAC5, in REAC5 the compound of formula (IV) is reacted with NH₂OH. The reaction product of REAC5 is a compound of formula (V); 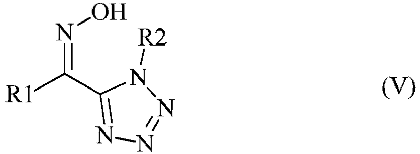 with R1 and R2 as defined above, also with all their embodiments.

ST6 comprises a reaction REAC6, in REAC6 the compound of formula (V) is reacted with a compound of formula (HETLIG); 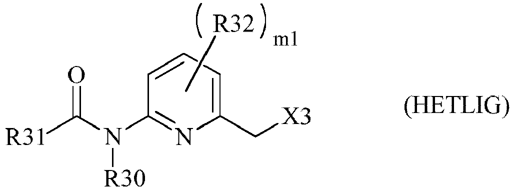
R30 is selected from the group consisting of H-C(O) and R40-C(O);
   R40 is selected from the group consisting of C₁₋₈ alkyl, C₁₋₆ alkoxy, phenyl, phenyloxy, and pyridyl;
   R40 can be unsubstituted or substituted by 1, 2 or 3 substitutents selected from the group consisting of CN, NO₂, F, Cl, Br, C₁₋₂ alkyl and C₁₋₂ alkoxy;
X3 is F, Cl, Br or I;
wherein R31, R32 and m1 are as defined herein, also with all their embodiments.

Preferably,
R30 is selected from the group consisting of methoxycarbonyl, ethoxycarbonyl, isopropoxycarbonyl, n-propoxycarbonyl, n-butoxycarbonyl, iso-butoxycarbonyl, sec-butoxycarbonyl, tert-butoxycarbonyl, formyl, acetyl, n-propylcarbonyl, iso-propylcarbonyl, n-butylcarbonyl, iso-butylcarbonyl, sec-butylcarbonyl, pivaloyl, octanoyl, benzoyl, 2,6-dimethoxybenzoyl, 3,5-nitrobenzoyl, 2,4,6-trichlorobenzoyl, 4-chlorobenzoyl and 2-pyridylcarbonyl;
R31 is selected from the group consisting of methyl, ethyl, n-propyl, iso-propyl, n-butyl, iso-butyl, sec-butyl, tert-butyl, n-pentyl, n-octyl, cyclopropyl, cyclobutyl, cyclopentyl, cyclohexyl, cycloheptyl, 2,2-dimethylcyclopropyl, menthyl, chloromethyl, fluoromethyl, trifluoromethyl, methoxymethyl, ethoxymethyl, methoxyethyl, methoxypropyl, ethoxybutyl, methoxybutyl, methoxyhexyl, propoxyoctyl, 2-methoxy-1,1-dimethylethyl, 1-ethoxy-1-methylethyl, carbomethoxymethyl, 1-carboethoxy-2,2-dimethyl-3-cyclopropyl, hydroxymethyl, hydroxyethyl, 1-hydroxypropyl, methoxy, ethoxy, n-propoxy, iso-propoxy, n-butoxy, iso-butoxy, sec-butoxy, tert-butoxy, n-pentyloxy, n-hexyloxy, n-decyloxy, cyclopropyloxy, cyclobutyloxy, cyclopentyloxy, cyclohexyloxy, menthyloxy, chloromethoxy, fluoromethoxy, trifluoromethoxy, methoxymethoxy, ethoxymethoxy, methoxyethoxy, 3-ethoxypropoxy, 2-ethoxybutoxy, 4-butoxybutoxy, 1-butoxypentoxy, fluoromethoxymethoxy, dichloromethoxymethoxy, 1 ,2-dibromo-3-methoxypropoxy and 3-isopropoxy-2-methylpropoxy;
R32 is selected from the group consisting of F, Cl, Br, CN, NO₂, OH, SH, C(O)H, COOH, NH₂, methylamino, dimethylamino, methylethylamino, diethylamino, tert-butoxycarbonylmethylamino, tert-butoxycarbonylamino, acetylmethylamino, acetylethylamino, benzoylmethylamino, methyl, ethyl, n-propyl, iso-propyl, n-butyl, iso-butyl, sec-butyl, tert-butyl, n-pentyl, n-octyl, cyclopropyl, cyclobutyl, cyclopentyl, cyclohexyl, cycloheptyl, 2,2-dimethylcyclopropyl, menthyl, chloromethyl, fluoromethyl, trifluoromethyl, methoxymethyl, ethoxymethyl, methoxyethyl, methoxypropyl, ethoxybutyl, methoxybutyl, methoxyhexyl, propoxyoctyl, 2-methoxy-1,1-dimethylethyl, 1-ethoxy-1-methylethyl, carbomethoxymethyl, 1-carboethoxy-2,2-dimethyl-3-cyclopropyl, hydroxymethyl, hydroxyethyl, 1-hydroxypropyl, phenyl, 1-naphthyl, 2-naphthyl, 6-methylphenyl, 4-methylphenyl, 4-fluorophenyl, 4-chlorophenyl, 2,4-dichlorophenyl, 3,4-dichlorophenyl, 3,5-dichlorophenyl, 2,6-difluorophenyl, 4-trifluoromethylphenyl, 4-methoxyphenyl, 3,4-dimethoxyphenyl, 3-phenoxyphenyl, 4-trifluoromethoxyphenyl, 4-methoxy-1-naphthyl, pyridinyl, pyridyl, pyrazolyl, imidazolyl, pyrrolidino, 3-trifluoromethylpyridin-2-yl, 4-trifluoromethoxy-2-pyridyl, 3-methyl-1-pyrazolyl, 4-trifluoromethyl-1-imidazolyl, 3,4-difluoropyrrolidino, methoxy, ethoxy, n-propoxy, iso-propoxy, n-butoxy, sec-butoxy, iso-butoxy, tert-butoxy, methoxymethoxy, ethoxymethoxy, methoxyethoxy, ethoxyethoxy, phenoxy, trichloromethoxy, trifluoromethoxy, difluoromethoxy, 2,2,2-trifluor-ethoxy and 2-fluoroethoxy,
S(O)ₘ₂R41, C(O)R41 and CO₂R41;
   R41 is selected from the group consisting of NH₂, methylamino, dimethylamino, methylethylamino, diethylamino, tert-butoxycarbonylmethylamino, tert-butoxycarbonylamino, acetylmethylamino, acetylethylamino, benzoylmethylamino, methyl, ethyl, n-propyl, iso-propyl, n-butyl, iso-butyl, sec-butyl, tert-butyl, n-pentyl, n-octyl, cyclopropyl, cyclobutyl, cyclopentyl, cyclohexyl, cycloheptyl, 2,2-dimethylcyclopropyl, menthyl, chloromethyl, fluoromethyl, trifluoromethyl, methoxymethyl, ethoxymethyl, methoxyethyl, methoxypropyl, ethoxybutyl, methoxybutyl, methoxyhexyl, propoxyoctyl, 2-methoxy-1,1-dimethylethyl, 1-ethoxy-1-methylethyl, carbomethoxymethyl, 1-carboethoxy-2,2-dimethyl-3-cyclopropyl, hydroxymethyl, hydroxyethyl, 1-hydroxypropyl, phenyl, 1-naphthyl, 2-naphthyl, 6-methylphenyl, 4-methylphenyl, 4-fluorophenyl, 4-chlorophenyl, 2,4-dichlorophenyl, 3,4-dichlorophenyl, 3,5-dichlorophenyl, 2,6-difluorophenyl, 4-trifluoromethylphenyl, 4-methoxyphenyl, 3,4-dimethoxyphenyl, 3-phenoxyphenyl, 4-trifluoromethoxyphenyl, 4-methoxy-1-naphthyl, pyridinyl, pyridyl, pyrazolyl, imidazolyl, pyrrolidino, 3-trifluoromethylpyridin-2-yl, 4-trifluoromethoxy-2-pyridyl, 3-methyl-1-pyrazolyl, 4-trifluoromethyl-1-imidazolyl and 3,4-difluoropyrrolidino, m2 is 0, 1 or 2;
m1 is 0, 1 or 2;
X3 is F, Cl or Br.

More preferably,
R30 is selected from the group consisting of benzoyl, 2,6-dimethoxybenzoyl, 3,5-nitrobenzoyl, 2,4,6-trichlorobenzoyl and 4-chlorobenzoyl;
R31 is tert-butoxy;
m1 is 0;
X3 is Cl or Br.

The reaction product of REAC6 is compound of formula (VI).

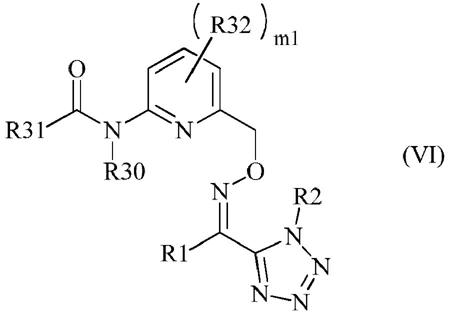

ST7 comprises a reaction REAC7, in REAC7 the compound of formula (VI) is reacted with a base BAS7.

Preferably, BAS7 is selected from the group consisting of alkali metal hydroxides, alkaline earth metal hydroxides, carbonates of alkali metal or of alkaline earth metal, hydrides of alkali metal or of alkaline earth metal, C₁₋₂ alkoxides of alkali metal or of alkaline earth metal, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
more preferably, BAS7 is selected from the group consisting of sodium hydroxide, potassium hydroxide, magnesium hydroxide, calcium hydroxide, sodium carbonate, potassium carbonate, magnesium carbonate, calcium carbonate, sodium hydride, calcium hydride, sodium methoxide, sodium ethoxide, magnesium methoxide, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
even more preferably, BAS7 is selected from the group consisting of sodium hydroxide, potassium hydroxide, magnesium hydroxide, calcium hydroxide, sodium carbonate, potassium carbonate, magnesium carbonate, calcium carbonate, sodium methoxide, sodium ethoxide, magnesium methoxide, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
especially, BAS7 is selected from the group consisting of sodium hydroxide, potassium hydroxide, sodium carbonate, triethylamine, diisopropylethylamine, pyridine, N,Ndimethylaminopyridine, 1,4-diazabicyclo[2.2.2]octane, 4-(dimethylamino)pyridine, 1,8-diazabicyclo[5.4.0]undec-7-ene, 1,5-diazabicyclo[4.3.0]non-5-ene, and mixtures thereof;
more especially, BAS7 is sodium hydroxide.

The reaction product of REAC7 is a compound of formula (VII).

### Examples

### Methods

1H NMR was done with triisobutylphosphate as internal standard, if not otherwise stated.

### Example 1: N-methyl 2-oxophenylthioacetamide

To thionyl chloride (0.725 ml, 10.0 mmol) were added compound of formula (I-1) (0.117 ml, 1.00 mmol) and pyridine (0.008 ml, 0.1 mmol), and the resulting mixture was stirred at room temperature for 18 h. The excess thionyl chloride was evaporated off (40 mbar), and the residue was redissolved in toluene (1.0 ml). The resulting solution was added dropwise at 10 °C to a stirred solution of methylamine in water (40% by weight, 0.9 ml, 10.4 mmol). After stirring at room temperature for 1 h 15 min, the mixture was diluted with IN aq HCl (10 ml), and extracted with ethyl acetate (3 ml). Concentration under reduced pressure yielded compound of formula (II-1) as an oil.
1H NMR showed that the yield was 61%.
¹H NMR (d6-DMSO, 400 MHz) delta = 11.11 (s, br, 1H), 7.91 (d, br, J = 8 Hz, 2H), 7.68 (t, br, J = 8 Hz, 1H), 7.55 (t, br, J = 8 Hz, 2H), 3.18 (d, J = 3 Hz, 3H).

### Example 2: N-methyl 2-oxophenylthioacetamide

To thionyl chloride (4.35 ml, 60.0 mmol) at 15 °C were added pyridine (0.04 ml, 0.50 mmol) and then compound of formula (I-1) (1.16 ml, 10.0 mmol), and the resulting mixture was stirred at room temperature for 19 h. The excess thionyl chloride was evaporated off (7 mbar), and the residue was redissolved in toluene (10.0 ml). The resulting solution was added portion wise to a stirred mixture of methylamine in water (40% by weight, 1.30 ml, 15 mmol), water (5.0 ml), and potassium carbonate (2.37 g, 17 mmol). After stirring at 0 °C for 5.5 h the mixture was diluted with cold IN aq HCl (60 ml), and extracted with ethyl acetate (3 times with 15 ml). The combined extracts were washed once with brine and dried (MgSO₄). Concentration under reduced pressure yielded compound of formula (II-1) as an oil. ¹H NMR showed that the yield was 74%.

### Example 3: 1-Methyl-5-benzoyltetrazole

To compound of formula (II-1) (90 mg with 80% being compound of formula (II-1), 0.40 mmol), prepared according to example 2, was added thionyl chloride (0.50 ml, 6.9 mmol). The mixture was stirred at room temperature. Analysis by ¹H NMR after 2 h indicated over 90% conversion to compound of formula (III-1).
After stirring for further 40 min the excess thionyl chloride was evaporated off. The residue was redissolved in toluene (1.0 ml) and the resulting solution was added drop wise at 0 °C to a solution of sodium azide (0.19 g, 3.0 mmol) in water (0.5 ml). The resulting mixture was stirred at 0 °C for 3 h, and then at room temperature for 1 h 20 min. Then the mixture was diluted with brine (10 ml), and the product extracted with ethyl acetate. Yield determination by ¹H NMR indicated a yield of 69% of compound of formula (IV-1).
¹H NMR (d₆-DMSO, 400 MHz) delta = 8.29 (d, br, J = 8 Hz, 2H), 7.80 (t, br, J = 8 Hz, 1H), 7.65 (t, br, J = 8 Hz, 2H), 4.31 (s, 3H).

### Example 4: 1-Methyl-5-benzoyltetrazole

To compound of formula (II-1) (90 mg with 80% being compound of formula (II-1), 0.40 mmol), prepared according to example 2, was added toluene (0.8 ml) and then thionyl chloride (0.109 ml, 1.51 mmol). The mixture was stirred at room temperature for 2 h 40 min, and the excess thionyl chloride was evaporated off. The residue was redissolved in toluene (1.0 ml) and the resulting solution was added dropwise at 0 °C to a solution of sodium azide (0.19 g, 3.0 mmol) in water (0.5 ml). The resulting mixture was stirred at 0 °C for 3 h, and then at room temperature for 1 h 20 min. Then the mixture was diluted with brine (10 ml), and the product extracted with ethyl acetate. Yield determination by ¹H NMR indicated a yield of 76% of compound of formula (IV-1).

## Claims

1. Method for the preparation of compound of formula (IV); 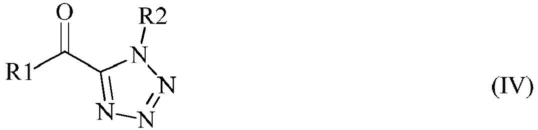 the method comprises four steps, a step ST1, a step ST2, a step ST3 and a step ST4;
ST4 comprises a reaction REAC4 of a compound of formula (III) with a compound AZID; 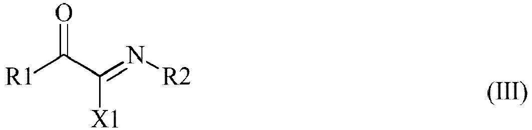
AZID is selected from the group consisting of alkali metal azide, alkali earth metal azide, [N(R10)(R11)(R12)R13]⁺ [N₃]⁻ and guanidinium azide;
R10, R11, R12 and R13 are identical or different and independently from each other selected from the group consisting of H and C₁₋₈ alkyl;
XI is selected from the group consisting of Cl, Br, S-CH₃, S-CH₂-CH₃, S-CH₂-Phenyl, S-C(O)-C(CH₃)₃, and SO₃H;
REAC4 results in compound of formula (IV);
compound of formula (III) is prepared in ST3;
ST3 comprises a reaction REAC3 of a compound of formula (II) with a compound COMPREAC3; 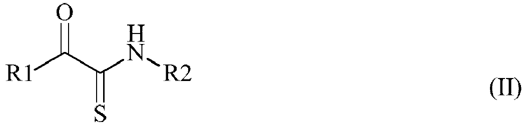 COMPREAC3 is selected from the group consisting of thionyl chloride, COCl₂, diphosgene, triphosgene, POCl₃, PCl₃, PCl₅, POBr₃, PBr₃, PBr₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, alkali metal persulfate salt, Cl₂, Br₂, and I₂;
REAC3 results in compound of formula (III);
wherein
R1 is selected from the group consisting of
phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy;
R2 is selected from the group consisting of
C₁₋₁₂ alkyl, the C₁₋₁₂ alkyl being unsubstituted or substituted by 1, 2 or 3 substituents selected from the group consisting of phenyl or 5 or 6 membered aromatic heterocycle with 1, 2 or 3 heteroatoms selected from the group consisting of N, O and S,
C₃₋₆ cycloalkyl,
phenyl, the phenyl being unsubstituted or substituted by 1, 2, 3, 4 or 5 substituents selected from the group consisting of halogen, nitro, cyano, trifluoromethyl, C₁₋₈ alkyl, and C₁₋₈ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₈ alkyl, and C₁₋₈ alkoxy;
compound of formula (II) is prepared in ST2;
ST1 comprises a reaction REAC1 of compound of formula (I) with SOCl₂; 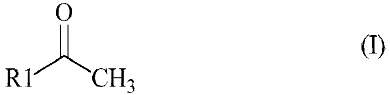 REAC1 results in a reaction product REACPROD1;
ST2 comprises a reaction REAC2 of REACPROD1 with R2-NH₂;
REAC2 results in compound of formula (II).

2. Method for the preparation of compound of formula (IV) according to claim 1, wherein
R1 is selected from the group consisting of
phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₆ alkyl, and C₁₋₆ alkoxy.

3. Method for the preparation of compound of formula (IV) according to claim 1 or 2,
wherein
R2 is selected from the group consisting of
C₁₋₆ alkyl, the C₁₋₆ alkyl being unsubstituted or substituted by 1 substituent selected from the group consisting of phenyl or 5 or 6 membered aromatic heterocycle with 1, 2 or 3 heteroatoms selected from the group consisting of N, O and S,
C₃₋₆ cycloalkyl,
phenyl, the phenyl being unsubstituted or substituted by 1, 2 or 3 substituents selected from the group consisting of halogen, nitro, cyano, trifluoromethyl, C₁₋₆ alkyl, and C₁₋₆ alkoxy, and
5 or 6 membered aromatic heterocycle with 1, 2 or 3 heteroatoms selected from the group consisting of N, O and S, the 5 or 6 membered aromatic heterocycle being unsubstituted or substituted by 1, 2 or 3 substituents selected from the group consisting of halogen, nitro, cyano, trifluoromethyl, C₁₋₆ alkyl, and C₁₋₆ alkoxy.

4. Method for the preparation of compound of formula (IV) according to one or more of claims 1 to 3, wherein
AZID is selected from the group consisting of alkali metal azide, and [N(R10)(R11)(R12)R13]⁺ [N₃]⁻.

5. Method for the preparation of compound of formula (IV) according to one or more of claims 1 to 4, wherein
XI is selected from the group consisting of Cl, S-CH₃, S-CH₂-CH₃, S-CH₂-Phenyl, S-C(O)-C(CH₃)₃, and SO₃H.

6. Method for the preparation of compound of formula (IV) according to one or more of claims 1 to 5, wherein
XI is Cl.

7. Method for the preparation of compound of formula (IV) according to one or more of claims 1 to 6, wherein
REAC4 is done in a solvent SOLV4; SOLV4 is selected from the group consisting of water, acetone, acetonitrile, ethanol, methanol, ethylene glycol, benzene, toluene, chlorobenzene, N,N-dimethylformamide, NMP, THF, dioxane, ethyl acetate, butyl acetate, and mixtures thereof.

8. Method for the preparation of compound of formula (IV) according to one or more of claims 1 to 7, wherein
REAC4 is done in the presence of a compound PTCS4, PTCS4 is a compound of formula [PTCS4];
[N(R20)(R21)(R22)R23]⁺[X2]⁻ [PTCS4]
R20, R21, R22 and R23 are identical or different and independently from each other selected from the group consisting of H, C₁₋₂₀ alkyl, phenyl and benzyl;
[X2]⁻ is selected from the group consisting of halide and hydrogensulfate.

9. Method for the preparation of compound of formula (IV) according to one or more of claims 1 to 8, wherein
COMPREAC3 is selected from the group consisting of thionyl chloride, COCl₂, diphosgene, triphosgene, POCl₃, PCl₃, PCl₅, S₂Cl₂, SCl₂, pivaloyl chloride, methyl chloride, methyl bromide, methyl iodide, ethyl chloride, ethyl bromide, ethyl iodide, benzyl chloride, benzyl bromide, dimethyl sulfate, diethyl sulfate, trimethylphosphate, triethylphosphate, methyl methanesulfonate, methyl benzenesulfonate, methyl tosylate, ethyl methanesulfonate, ethyl benzenesulfonate, ethyl tosylate, hydrogen peroxide, C₁₋₄ alkyl hydro peroxide, C₁₋₆ alkaloyl peroxide, perbenzoic acid, 3-chloro-perbenzoic acid, sodium persulfate salt, potassium persulfate salt, Cl₂, Br₂, and I₂.

10. Method for the preparation of compound of formula (IV) according to one or more of claims 1 to 9, wherein
COMPREAC3 is thionyl chloride.

11. Method for the preparation of compound of formula (IV) according to one or more of claims 1 to 10, wherein
R1 is phenyl; and
R2 is methyl.

12. Method for the preparation of compound of formula (VII), 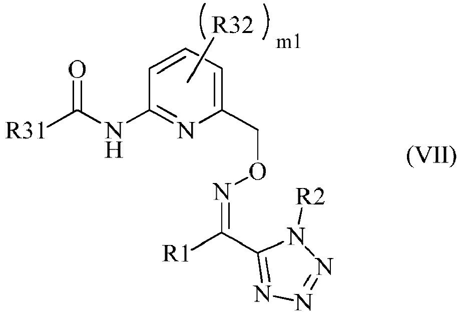 wherein the method comprises the four steps ST1, ST2, ST3 and ST4 as defined in one or more of claims 1 to 11;
R1 and R2 are as defined in anyone of claims 1 to 3;
R31 is selected from the group consisting of linear, branched and cyclic C₁₋₁₀ alkyl and linear, branched and cyclic C₁₋₁₀ alkoxy, wherein the linear, branched and cyclic C₁₋₁₀ alkyl and the linear, branched and cyclic C₁₋₁₀ alkoxy can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH;
R32 is selected from the group consisting of F, Cl, Br, CN, NO₂, OH, SH, C(O)H, COOH, N(R50)R51,
linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH,
phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy,
pyridinyl, pyridyl, pyrazolyl, imidazolyl, pyrrolidino, wherein the pyridinyl and the pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy,
C₁₋₄ alkoxy, the C₁₋₄ alkoxy being unsubstituted or substituted by 1, 2 or 3 substituents selected from the group consisting of C₁₋₂ alkoxy, F and Cl,
phenoxy,
S(O)ₘ₂R41, C(O)R41 and CO₂R41;
R41 is selected from the group consisting of
N(R50)R51,
linear, branched and cyclic C₁₋₁₀ alkyl, wherein the linear, branched and cyclic C₁₋₁₀ alkyl can be unsubstituted or substituted by 1, 2 or 3 substituents selected from the group consisting of F, Cl, Br, C₁₋₄ alkoxy, C₁₋₄ alkoxy substituted by F, Cl, difluoro, dichloro, bromo or dibromo, carbomethoxy, carboethoxy and OH,
phenyl, naphthyl, wherein the phenyl and the napthyl are unsubstituted or substituted by 1, 2 or 3 substituents selected from the group consisting of C₁₋₄ alkyl, F, Cl, trifluoromethyl, C₁₋₄ alkoxy, phenoxy and trifluoromethoxy,
pyridinyl, pyridyl, pyrazolyl, imidazolyl, pyrrolidino, wherein the pyridinyl and pyridyl and the pyrazolyl and the imidazolyl and the pyrrolidino are unsubstituted or substitutued by 1, 2 or 3 substituents selected from the group consisting of C₁₋₄ alkyl, F, Cl, trifluoromethyl, C₁₋₄ alkoxy and trifluoromethoxy,
m2 is 0, 1 or 2;
R50 and R51 are identical or different and selected from the group consisting of H, C₁₋₂ alkyl, C₁₋₄ alkoxycarbonyl and benzoyl;
m1 is 0, 1, 2 or 3;
when m1 is 2 or 3, then the substituents R32 can be identical or different from each other.

13. Method for the preparation of compound of formula (VII) according to claim 12, wherein
R1 is phenyl;
R2 is methyl;
R31 is tert-butoxy;
m1 is 0.

## Patentansprüche

1. Verfahren zur Herstellung einer Verbindung der Formel (IV) 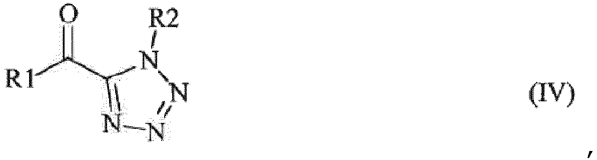 wobei das Verfahren vier Schritte, einen Schritt ST1, einen Schritt ST2, einen Schritt ST3 und einen Schritt ST4, umfasst,
ST4 eine Reaktion REAC4 einer Verbindung der Formel (III) mit einer Verbindung AZID umfasst, 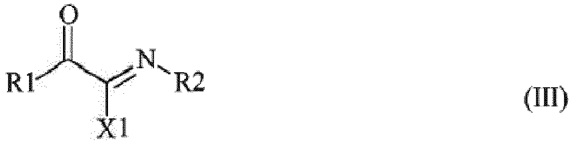
AZID aus der aus Alkaliazid, Erdalkaliazid, [N (R10) (R11) (R12) R34]⁺ [N₃]⁻ und Guanidiniumazid bestehenden Gruppe ausgewählt ist,
R10, R11, R12 und R13 gleich oder verschieden sind und unabhängig voneinander aus der aus H und C₁₋₈-Alkyl bestehenden Gruppe ausgewählt sind,
X1 aus der aus Cl, Br, S-CH₃, S-CH₂-CH₃-, S-CH₂-Phenyl, S-C(O)-C(CH₃)₃ und SO₃H bestehenden Gruppe ausgewählt ist,
REAC4 zu einer Verbindung der Formel (IV) führt,
die Verbindung der Formel (III) in ST3 dargestellt wird,
ST3 eine Reaktion REAC3 einer Verbindung der Formel (II) mit einer Verbindung COMPREAC3 umfasst, 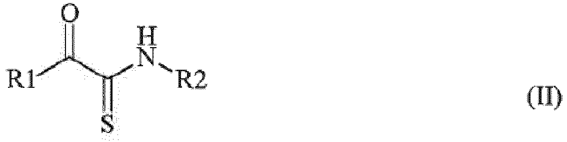 COMPREAC3 aus der aus Thionylchlorid, COCl₂, Diphosgen, Triphosgen, POCl₃, PCl₃, PCl₅, POBr₃, PBr₃, PBr₅, S₂Cl₂, SCl₂, Pivaloylchlorid, Methylchlorid, Methylbromid, Methyliodid, Ethylchlorid, Ethylbromid, Ethyliodid, Benzylchlorid, Benzylbromid, Dimethylsulfat, Diethylsulfat, Trimethylphosphat, Triethylphosphat, Methansulfonsäuremethylester, Benzolsulfonsäuremethylester, Toluolsulfonsäuremethylester, Methansulfonsäureethylester, Benzolsulfonsäureethylester, Toluolsulfonsäureethylester, Wasserstoffperoxid, C₁₋₄-Alkylhydroperoxid, C₁₋₆-Alkaloylperoxid, Perbenzoesäure, 3-Chlorperbenzoesäure, Alkalimetallpersulfatsalz, Cl₂, Br₂ und I₂ bestehenden Gruppe ausgewählt ist,
REAC3 zu einer Verbindung der Formel (III) führt,
wobei
R1 aus der aus
Phenyl, wobei das Phenyl unsubstituiert oder durch 1, 2, 3, 4 oder 5 aus der aus Halogen, Nitro, Cyano, Trifluormethyl, C₁₋₈-Alkyl und C₁₋₈-Alkoxy bestehenden Gruppe ausgewählte Substituenten substituiert ist, und
einem 5- oder 6-gliedrigen aromatischen Heterocyclus mit 1, 2 oder 3 aus der aus N, O und S bestehenden Gruppe ausgewählten Heteroatomen, wobei der 5- oder 6-gliedrige aromatische Heterocyclus unsubstituiert oder durch 1, 2 oder 3 aus der aus Halogen, Nitro, Cyano, Trifluormethyl, C₁₋₈-Alkyl und C₁₋₈-Alkoxy bestehenden Gruppe ausgewählte Substituenten substituiert ist,
bestehenden Gruppe ausgewählt ist,
R2 aus der aus
C₁₋₁₂-Alkyl, wobei das C₁₋₁₂-Alkyl unsubstituiert oder durch 1, 2 oder 3 aus der aus Phenyl und einem 5- oder 6-gliedrigen aromatischen Heterocyclus mit 1, 2 oder 3 aus der aus N, O und S bestehenden Gruppe ausgewählten Heteroatomen bestehenden Gruppe ausgewählte Substituenten substituiert ist,
C₃₋₆-Cycloalkyl,
Phenyl, wobei das Phenyl unsubstituiert oder durch 1, 2, 3, 4 oder 5 aus der aus Halogen, Nitro, Cyano, Trifluormethyl, C₁₋₈-Alkyl und C₁₋₈-Alkoxy bestehenden Gruppe ausgewählte Substituenten substituiert ist, und
einem 5- oder 6-gliedren aromatischen Heterocyclus mit 1, 2 oder 3 aus der aus N, O und S bestehenden Gruppe ausgewählten Heteroatomen, wobei der 5- oder 6-gliedrige aromatische Heterocyclus unsubstituiert oder durch 1, 2 oder 3 aus der aus Halogen, Nitro, Cyano, Trifluormethyl, C₁₋₈-Alkyl und C₁₋₈-Alkoxy bestehenden Gruppe ausgewählte Substituenten substituiert ist,
bestehenden Gruppe ausgewählt ist,
die Verbindung der Formel (II) in ST2 dargestellt wird,
ST1 eine Reaktion REAC1 einer Verbindung der Formel (I) mit SOCl₂ umfasst, 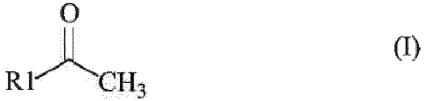
REAC1 zu einem Reaktionsprodukt REACPROD1 führt,
ST2 eine Reaktion REAC2 von REACPROD1 mit R2-NH₂ umfasst,
REAC2 zu einer Verbindung der Formel (II) führt.

2. Verfahren zur Herstellung einer Verbindung der Formel (IV) nach Anspruch 1, wobei
R1 aus der aus
Phenyl, wobei das Phenyl unsubstituiert oder durch 1, 2 oder 3 aus der aus Halogen, Nitro, Cyano, Trifluormethyl, C₁₋₆-Alkyl und C₁₋₆-Alkoxy bestehenden Gruppe ausgewählte Substituenten substituiert ist, und
einem 5- oder 6-gliedrigen aromatischen Heterocyclus mit 1, 2 oder 3 aus der aus N, O und S bestehenden Gruppe ausgewählten Heteroatomen, wobei der 5- oder 6-gliedrige aromatische Heterocyclus unsubstituiert oder durch 1, 2 oder 3 aus der aus Halogen, Nitro, Cyano, Trifluormethyl, C₁₋₆-Alkyl und C₁₋₆-Alkoxy bestehenden Gruppe ausgewählte Substituenten substituiert ist,
bestehenden Gruppe ausgewählt ist.

3. Verfahren zur Herstellung einer Verbindung der
Formel (IV) nach Anspruch 1 oder 2, wobei R2 aus der aus
C₁₋₆-Alkyl, wobei das C₁₋₆-Alkyl unsubstituiert oder durch 1 Substituenten ausgewählt aus der Gruppe bestehend aus Phenyl und einem 5- oder 6-gliedrigen aromatischen Heterocyclus mit 1, 2 oder 3 aus der aus N, O und S bestehenden Gruppe ausgewählten Heteroatomen substituiert ist,
C₃₋₆-Cycloalkyl,
Phenyl, wobei das Phenyl unsubstituiert oder durch 1, 2 oder 3 aus der aus Halogen, Nitro, Cyano, Trifluormethyl, C₁₋₆-Alkyl und C₁₋₆-Alkoxy bestehenden Gruppe ausgewählte Substituenten substituiert ist, und
einem 5- oder 6-gliedrigen aromatischen Heterocyclus mit 1, 2 oder 3 aus der aus N, O und S bestehenden Gruppe ausgewählten Heteroatomen, wobei der 5- oder 6-gliedrige aromatische Heterocyclus unsubstituiert oder durch 1, 2 oder 3 aus der aus Halogen, Nitro, Cyano, Trifluormethyl, C₁₋₆-Alkyl und C₁₋₆-Alkoxy bestehenden Gruppe ausgewählte Substituenten substituiert ist,
bestehenden Gruppe ausgewählt ist.

4. Verfahren zur Herstellung einer Verbindung der Formel (IV) nach einem oder mehreren der Ansprüche 1 bis 3, wobei
AZID aus der aus Alkaliazid und [N(R10) (R11) (R12)R13]⁺[N₃]⁻ bestehenden Gruppe ausgewählt ist.

5. Verfahren zur Herstellung einer Verbindung der Formel (IV) nach einem oder mehreren der Ansprüche 1 bis 4, wobei
X1 aus der aus Cl, S-CH₃, S-CH₂-CH₃, S-CH₂-Phenyl, S-C(O)-C(CH₃)₃ und SO₃H bestehenden Gruppe ausgewählt ist.

6. Verfahren zur Herstellung einer Verbindung der Formel (IV) nach einem oder mehreren der Ansprüche 1 bis 5, wobei
X1 für Cl steht.

7. Verfahren zur Herstellung einer Verbindung der Formel (IV) nach einem oder mehreren der Ansprüche 1 bis 6, wobei
REAC4 in einem Lösungsmittel SOLV4 durchgeführt wird; SOLV4 aus der aus Wasser, Aceton, Acetonitril, Ethanol, Methanol, Ethylenglykol, Benzol, Toluol, Chlorbenzol, N,N-Dimethylformamid, NMP, THF, Dioxan, Essigsäureethylester, Essigsäurebutylester und Mischungen davon bestehenden Gruppe ausgewählt ist.

8. Verfahren zur Herstellung einer Verbindung der Formel (IV) nach einem oder mehreren der Ansprüche 1 bis 7, wobei
REAC4 in Gegenwart einer Verbindung PTCS4 durchgeführt wird, es sich bei PTCS4 um eine Verbindung der Formel [PTCS4] handelt,
[N(R20) (R21) (R22)R23]⁺[X2]⁻ [PTCS4],
R20, R21, R22 und R23 gleich oder verschieden sind und unabhängig voneinander aus der aus H, C₁₋₂₀-Alkyl, Phenyl und Benzyl bestehenden Gruppe ausgewählt sind,
[X2]⁻ aus der aus Halogenid und Hydrogensulfat bestehenden Gruppe ausgewählt ist.

9. Verfahren zur Herstellung einer Verbindung der Formel (IV) nach einem oder mehreren der Ansprüche 1 bis 8, wobei
COMPREAC3 aus der aus Thionylchlorid, COCl₂, Diphosgen, Triphosgen, POCl₃, PCl₃, PCl₅, S₂Cl₂, SCl₂, Pivaloylchlorid, Methylchlorid, Methylbromid, Methyliodid, Ethylchlorid, Ethylbromid, Ethyliodid, Benzylchlorid, Benzylbromid, Dimethylsulfat, Diethylsulfat, Trimethylphosphat, Triethylphosphat, Methansulfonsäuremethylester, Benzolsulfonsäuremethylester, Toluolsulfonsäuremethylester, Methansulfonsäureethylester, Benzolsulfonsäureethylester, Toluolsulfonsäureethylester, Wasserstoffperoxid, C₁₋₄-Alkylhydroperoxid, C₁₋₆-Alkaloylperoxid, Perbenzoesäure, 3-Chlorperbenzoesäure, Natriumpersulfatsalz, Kaliumpersulfatsalz, Cl₂, Br₂ und I₂ bestehenden Gruppe ausgewählt ist.

10. Verfahren zur Herstellung einer Verbindung der Formel (IV) nach einem oder mehreren der Ansprüche 1 bis 9, wobei
COMPREAC3 für Thionylchlorid steht.

11. Verfahren zur Herstellung einer Verbindung der Formel (IV) nach einem oder mehreren der Ansprüche 1 bis 10, wobei
R1 für Phenyl steht und
R2 für Methyl steht.

12. Verfahren zur Herstellung einer Verbindung der Formel (VII) 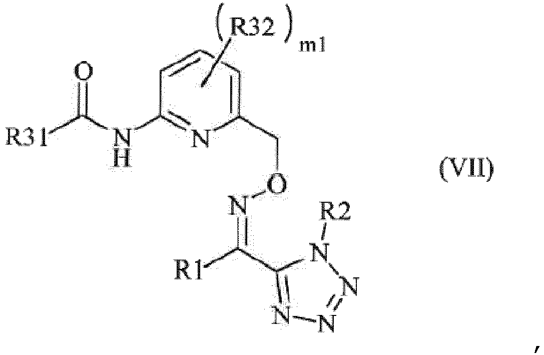 wobei das Verfahren die wie in einem oder mehreren der Ansprüche 1 bis 11 definierten vier Schritte ST1, ST2, ST3 und ST4 umfasst,
R1 und R2 wie in einem der Ansprüche 1 bis 3 definiert sind,
R31 aus der aus geradkettigem, verzweigtem und cyclischem C₁₋₁₀-Alkyl und geradkettigem, verzweigtem und cyclischem C₁₋₁₀-Alkoxy bestehenden Gruppe ausgewählt ist, wobei das geradkettige, verzweigte und cyclische C₁₋₁₀-Alkyl und das geradkettige, verzweigte und cyclische C₁₋₁₀-Alkoxy unsubstituiert oder durch 1, 2 oder 3 aus der aus F, Cl, Br, C₁₋₄-Alkoxy, C₁₋₄-Alkoxy substituiert durch F, Cl, Difluor, Dichlor, Brom oder Dibrom, Carbomethoxy, Carboethoxy und OH bestehenden Gruppe ausgewählte Substituenten substituiert sein kann,
R32 aus der aus F, Cl, Br, CN, NO₂, OH, SH, C(O)H, COOH, N(R50)R51,
geradkettigem, verzweigtem und cyclischem C₁₋₁₀-Alkyl, wobei das geradkettige, verzweigte und cyclische C₁₋₁₀-Alkyl unsubstituiert oder durch 1, 2 oder 3 aus der aus F, Cl, Br, C₁₋₄-Alkoxy, C₁₋₄-Alkoxy substituiert durch F, Cl, Difluor, Dichlor, Brom oder Dibrom, Carbomethoxy, Carboethoxy und OH bestehenden Gruppe ausgewählte Substituenten substituiert sein kann,
Phenyl, Naphthyl, wobei das Phenyl und das Naphthyl unsubstituiert oder durch 1, 2 oder 3 aus der aus C₁₋₄-Alkyl, F, Cl, Trifluormethyl, C₁₋₄-Alkoxy, Phenoxy und Trifluormethoxy ausgewählte Substituenten substituiert sind,
Pyridinyl, Pyridyl, Pyrazolyl, Imidazolyl, Pyrrolidino, wobei das Pyridinyl und das Pyridyl und das Pyrazolyl und das Imidazolyl und das Pyrrolidino unsubstituiert oder durch 1, 2 oder 3 aus der aus C₁₋₄-Alkyl, F, Cl, Trifluormethyl, C₁₋₄-Alkoxy und Trifluormethoxy bestehenden Gruppe ausgewählte Substituenten substituiert sind,
C₁₋₄-Alkoxy, wobei das C₁₋₄-Alkoxy unsubstituiert oder durch 1, 2 oder 3 aus der aus C₁₋₂-Alkoxy, F und Cl bestehenden Gruppe ausgewählte Substituenten substituiert ist,
Phenoxy,
S(O)ₘ₂R41, C(O)R41 und CO₂R41
bestehenden Gruppe ausgewählt ist,
R41 aus der aus
N(R50)R51,
geradkettigem, verzweigtem und cyclischem C₁₋₁₀-Alkyl, wobei das geradkettige, verzweigte und cyclische C₁₋₁₀-Alkyl unsubstituiert oder durch 1, 2 oder 3 aus der aus F, Cl, Br, C₁₋₄-Alkoxy, C₁₋₄-Alkoxy substituiert durch F, Cl, Difluor, Dichlor, Brom oder Dibrom, Carbomethoxy, Carboethoxy und OH bestehenden Gruppe ausgewählte Substituenten substituiert sein kann,
Phenyl, Naphthyl, wobei das Phenyl und das Naphthyl unsubstituiert oder durch 1, 2 oder 3 aus der aus C₁₋₄-Alkyl, F, Cl, Trifluormethyl, C₁₋₄-Alkoxy, Phenoxy und Trifluormethoxy bestehenden Gruppe ausgewählte Substituenten substituiert sind,
Pyridinyl, Pyridyl, Pyrazolyl, Imidazolyl, Pyrrolidino, wobei das Pyridinyl und Pyridyl und das Pyrazolyl und das Imidazolyl und das Pyrrolidino unsubstituiert oder durch 1, 2 oder 3 aus der aus C₁₋₄-Alkyl, F, Cl, Trifluormethyl, C₁₋₄-Alkoxy und Trifluormethoxy bestehenden Gruppe ausgewählte Substituenten substituiert sind
bestehenden Gruppe ausgewählt ist,
m2 für 0, 2 oder 2 steht,
R50 und R51 gleich oder verschieden sind und aus der aus H, C₁₋₂-Alkyl, C₁₋₄-Alkoxycarbonyl und Benzoyl bestehenden Gruppe ausgewählt sind,
m1 für 0, 1, 2 oder 3 steht,
wenn m1 für 2 oder 3 steht, die Substituenten R32 gleich oder verschieden voneinander sein können.

13. Verfahren zur Herstellung einer Verbindung der Formel (VII) nach Anspruch 12, wobei
R1 für Phenyl steht,
R2 für Methyl steht,
R31 für tert.-Butoxy steht,
m1 für 0 steht.

## Revendications

1. Procédé de préparation d'un composé de formule (IV) ; 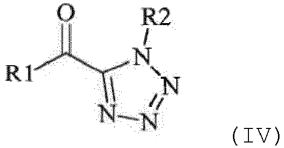 le procédé comprenant quatre étapes, une étape ST1, une étape ST2, une étape ST3 et une étape ST4 ;
ST4 comprend une réaction REAC4 d'un composé de formule (III) avec un composé AZID ; 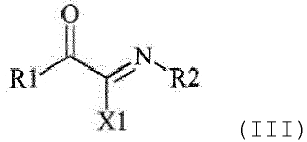 AZID est choisi dans le groupe constitué d'un azide de métal alcalin, un azide de métal alcalino-terreux, [N(R10) (R11) (R12)R13]⁺[N₃]⁻ et l'azide de guanidinium ;
R10, R11, R12 et R13 sont identiques ou différents et, indépendamment les uns des autres, choisis dans le groupe constitué de H et alkyle en C₁₋₈;
X1 est choisi dans le groupe constitué de Cl, Br, S-CH₃, S-CH₂-CH₃, S-CH₂-phényle, S-C(O)-C(CH₃)₃, et SO₃H ;
REAC4 conduit à un composé de formule (IV) ;
le composé de formule (III) est préparé dans ST3 ;
ST3 comprend une réaction REAC3 d'un composé de formule (II) avec un composé COMPREAC3 ; 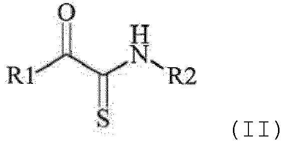 COMPREAC3 est choisi dans le groupe constitué des chlorure de thionyle, COCl₂, diphosgène, triphosgène, POCl₃, PCl₃, PCl₅, POBr₃, PBr₃, PBr₅, S₂Cl₂, SCl₂, chlorure de pivaloyle, chlorure de méthyle, bromure de méthyle, iodure de méthyle, chlorure d'éthyle, bromure d'éthyle, iodure d'éthyle, chlorure de benzyle, bromure de benzyle, sulfate de diméthyle, sulfate de diéthyle, phosphate de triméthyle, phosphate de triéthyle, méthanesulfonate de méthyle, benzènesulfonate de méthyle, tosylate de méthyle, méthanesulfonate d'éthyle, benzènesulfonate d'éthyle, tosylate d'éthyle, peroxyde d'hydrogène, hydroperoxyde d'alkyle en C₁₋₄, peroxyde d'alcaloyle en C₁₋₆, acide perbenzoïque, acide 3-chloro-perbenzoïque, sel de persulfate de métal alcalin, Cl₂, Br₂ et I₂ ; REAC3 conduit à un composé de formule (III) ;
dans lequel
R1 est choisi dans le groupe constitué de phényle, le phényle étant non substitué ou substitué par 1, 2, 3, 4 ou 5 substituants choisis dans le groupe constitué d'halogène, nitro, cyano, trifluorométhyle, alkyle en C₁₋₈et alcoxy en C₁₋₈, et
hétérocycle aromatique de 5 ou 6 chaînons avec 1, 2 ou 3 hétéroatomes choisis dans le groupe constitué de N, O et S, l'hétérocycle aromatique de 5 ou 6 chaînons étant non substitué ou substitué par 1, 2 ou 3 substituants choisis dans le groupe constitué d'halogène, nitro, cyano, trifluorométhyle, alkyle en C₁₋₈ et alcoxy en C₁₋₈ ; R2 est choisi dans le groupe constitué de alkyle en C₁₋₁₂, l'alkyle en C₁₋₂ étant non substitué ou substitué par 1, 2 ou 3 substituants choisis dans le groupe constitué de phényle ou hétérocycle aromatique de 5 ou 6 chaînons avec 1, 2 ou 3 hétéroatomes choisis dans le groupe constitué de N, O et S,
cycloalkyle en C₃₋₆,
phényle, le phényle étant non substitué ou substitué par 1, 2, 3, 4 ou 5 substituants choisis dans le groupe constitué d'halogène, nitro, cyano, trifluorométhyle, alkyle en C₁₋₈ et alcoxy en C₁₋₈, et
hétérocycle aromatique de 5 ou 6 chaînons avec 1, 2 ou 3 hétéroatomes choisis dans le groupe constitué de N, O et S, l'hétérocycle aromatique de 5 ou 6 chaînons étant non substitué ou substitué par 1, 2 ou 3 substituants choisis dans le groupe constitué d'halogène, nitro, cyano, trifluorométhyle, alkyle en C₁₋₈ et alcoxy en C₁₋₈ ; le composé de formule (II) est préparé dans ST2 ;
ST1 comprend une réaction REAC1 d'un composé de formule (I) avec SOCl₂ ; 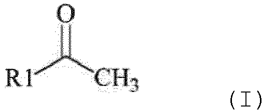 REAC1 conduit à un produit de réaction REACPROD1 ;
ST2 comprend une réaction REAC2 de REACPROD1 avec R2-NH₂ ;
REAC2 conduit à un composé de formule (II).

2. Procédé de préparation d'un composé de formule (IV) selon la revendication 1, dans lequel R1 est choisi dans le groupe constitué de phényle, le phényle étant non substitué ou substitué par 1, 2 ou 3 substituants choisis dans le groupe constitué d'halogène, nitro, cyano, trifluorométhyle, alkyle en C₁₋₆, et alcoxy en C₁₋₆, et
hétérocycle aromatique de 5 ou 6 chaînons avec 1, 2 ou 3 hétéroatomes choisis dans le groupe constitué de N, O et S, l'hétérocycle aromatique de 5 ou 6 chaînons étant non substitué ou substitué par 1, 2 ou 3 substituants choisis dans le groupe constitué d'halogène, nitro, cyano, trifluorométhyle, alkyle en C₁₋₆, et alcoxy en C₁₋₆.

3. Procédé de préparation d'un composé de formule (IV) selon la revendication 1 ou 2,
dans lequel
R2 est choisi dans le groupe constitué de alkyle en C₁₋₆, l'alkyle en C₁₋₆ étant non substitué ou substitué par 1 substituant choisi dans le groupe constitué de phényle ou hétérocycle aromatique de 5 ou 6 chaînons avec 1, 2 ou 3 hétéroatomes choisis dans le groupe constitué de N, O et S,
cycloalkyle en C₃₋₆,
phényle, le phényle étant non substitué ou substitué par 1, 2 ou 3 substituants choisis dans le groupe constitué d'halogène, nitro, cyano, trifluorométhyle, alkyle en C₁₋₆ et alcoxy en C₁₋₆, et
hétérocycle aromatique de 5 ou 6 chaînons avec 1, 2 ou 3 hétéroatomes choisis dans le groupe constitué de N, O et S, l'hétérocycle aromatique de 5 ou 6 chaînons étant non substitué ou substitué par 1, 2 ou 3 substituants choisis dans le groupe constitué d'halogène, nitro, cyano, trifluorométhyle, alkyle en C₁₋₆, et alcoxy en C₁₋₆.

4. Procédé de préparation d'un composé de formule (IV) selon une ou plusieurs des revendications 1 à 3, dans lequel
AZID est choisi dans le groupe constitué d'un azide de métal alcalin et [N(R10) (R11) (R12)R13]⁺[N₃]⁻.

5. Procédé de préparation d'un composé de formule (IV) selon une ou plusieurs des revendications 1 à 4, dans lequel
X1 est choisi dans le groupe constitué de Cl, S-CH₃, S-CH₂-CH₃, S-CH₂-phényle, S-C(O)-C(CH₃)₃ et SO₃H.

6. Procédé de préparation d'un composé de formule (IV) selon une ou plusieurs des revendications 1 à 5, dans lequel
X1 est Cl.

7. Procédé de préparation d'un composé de formule (IV) selon une ou plusieurs des revendications 1 à 6, dans lequel
REAC4 est conduite dans un solvant SOLV4 ; SOLV4 est choisi dans le groupe constitué de l'eau, l'acétone, l'acétonitrile, l'éthanol, le méthanol, l'éthylène glycol, le benzène, le toluène, le chlorobenzène, le N,N-diméthylformamide, NMP, THF, le dioxane, l'acétate d'éthyle, l'acétate de butyle, et des mélanges de ceux-ci.

8. Procédé de préparation d'un composé de formule (IV) selon une ou plusieurs des revendications 1 à 7, dans lequel
REAC4 est conduit en présence d'un composé PTCS4, PTCS4 est un composé de formule [PTCS4] ;
[N(R20)(R21)(R22)R23]⁺[X2]⁻ [PTCS4]
R20, R21, R22 et R23 sont identiques ou différents et, indépendamment les uns des autres, choisis dans le groupe constitué de H, alkyle en C₁₋₂₀, phényle et benzyle ; [X2]⁻ est choisi dans le groupe constitué d'halogénure et hydrogénosulfate.

9. Procédé de préparation d'un composé de formule (IV) selon une ou plusieurs des revendications 1 à 8, dans lequel
COMPREAC3 est choisi dans le groupe constitué des chlorure de thionyle, COCl₂, diphosgène, triphosgène, POCl₃, PCl₃, PCl₅, S₂Cl₂, SCl₂, chlorure de pivaloyle, chlorure de méthyle, bromure de méthyle, iodure de méthyle, chlorure d'éthyle, bromure d'éthyle, iodure d'éthyle, chlorure de benzyle, bromure de benzyle, sulfate de diméthyle, sulfate de diéthyle, phosphate de triméthyle, phosphate de triéthyle, méthanesulfonate de méthyle, benzènesulfonate de méthyle, tosylate de méthyle, méthanesulfonate d'éthyle, benzènesulfonate d'éthyle, tosylate d'éthyle, peroxyde d'hydrogène, hydroperoxyde d'alkyle en C₁₋₄, peroxyde d'alcaloyle en C₁₋₆, acide perbenzoïque, acide 3-chloro-perbenzoïque, sel de persulfate de sodium, sel de persulfate de potassium, Cl₂, Br₂ et I₂.

10. Procédé de préparation d'un composé de formule (IV) selon une ou plusieurs des revendications 1 à 9, dans lequel
COMPREAC3 est le chlorure de thionyle.

11. Procédé de préparation d'un composé de formule (IV) selon une ou plusieurs des revendications 1 à 10, dans lequel
R1 est phényle ; et
R2 est méthyle.

12. Procédé de préparation d'un composé de formule (VII), 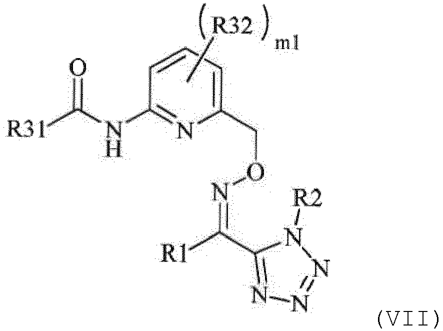 le procédé comprenant les quatre étapes ST1, ST2, ST3 et ST4 telles que définies dans une ou plusieurs des revendications 1 à 11 ;
R1 et R2 sont tels que définis dans l'une quelconque des revendications 1 à 3 ;
R31 est choisi dans le groupe constitué d'alkyle en C₁₋₁₀ linéaire, ramifié et cyclique et alcoxy en C₁₋₁₀ linéaire, ramifié et cyclique, où l'alkyle en C₁₋₁₀ linéaire, ramifié et cyclique et l'alcoxy en C₁₋₁₀ linéaire, ramifié et cyclique peuvent être non substitués ou substitués par 1, 2 ou 3 substituants choisis dans le groupe constitué de F, Cl, Br, alcoxy en C₁₋₄, alcoxy en C₁₋₄ substitué par F, Cl, difluoro, dichloro, bromo ou dibromo, carbométhoxy, carboéthoxy et OH ;
R32 est choisi dans le groupe constitué de F, Cl, Br, CN, NO₂, OH, SH, C(O)H, COOH, N(R50)R51,
alkyle en C₁₋₁₀ linéaire, ramifié et cyclique, où l'alkyle en C₁₋₁₀ linéaire, ramifié et cyclique peut être non substitué ou substitué par 1, 2 ou 3 substituants choisis dans le groupe constitué de F, Cl, Br, alcoxy en C₁₋₄, alcoxy en C₁₋₄ substitué par F, Cl, difluoro, dichloro, bromo ou dibromo, carbométhoxy, carboéthoxy et OH, phényle, naphtyle, où le phényle et le naphtyle sont non substitués ou substitués par 1, 2 ou 3 substituants choisis dans le groupe constitué d'alkyle en C₁₋₄, F, Cl, trifluorométhyle, alcoxy en C₁₋₄, phénoxy et trifluorométhoxy, pyridinyle, pyridyle, pyrazolyle, imidazolyle, pyrrolidino, où les pyridinyle et pyridyle et pyrazolyle et imidazolyle et pyrrolidino sont non substitués ou substitués par 1, 2 ou 3 substituants choisis dans le groupe constitué d'alkyle en C₁₋₄, F, Cl, trifluorométhyle, alcoxy en C₁₋₄ et trifluorométhoxy, alcoxy en C₁₋₄, l'alcoxy en C₁₋₄ étant non substitué ou substitué par 1, 2 ou 3 substituants choisis dans le groupe constitué d'alcoxy en C₁₋₂, F et Cl, phénoxy,
S(O)ₘ₂R41, C(O)R41 et CO₂R41 ;
R41 est choisi dans le groupe constitué de N(R50)R51,
alkyle en C₁₋₁₀ linéaire, ramifié et cyclique, où l'alkyle en C₁₋₁₀ linéaire, ramifié et cyclique peut être non substitué ou substitué par 1, 2 ou 3 substituants choisis dans le groupe constitué de F, Cl, Br,
alcoxy en C₁₋₄, alcoxy en C₁₋₄ substitué par F, Cl, difluoro, dichloro, bromo ou dibromo, carbométhoxy, carboéthoxy et OH,
phényle, naphtyle, où le phényle et le napthyle sont non substitués ou substitués par 1, 2 ou 3 substituants choisis dans le groupe constitué d'alkyle en C₁₋₄, F, Cl, trifluorométhyle, alcoxy en C₁₋₄, phénoxy et trifluorométhoxy,
pyridinyle, pyridyle, pyrazolyle, imidazolyle, pyrrolidino, où les pyridinyle et pyridyle et pyrazolyle et imidazolyle et pyrrolidino sont non substitués ou substitués par 1, 2 ou 3 substituants choisis dans le groupe constitué d'alkyle en C₁₋₄, F, Cl, trifluorométhyle, alcoxy en C₁₋₄ et trifluorométhoxy,
m2 est 0, 1 ou 2 ;
R50 et R51 sont identiques ou différents et choisis dans le groupe constitué de H, alkyle en C₁₋₂, (alcoxy en C₁₋₄) carbonyle et benzoyle ;
m1 est 0, 1, 2 ou 3 ;
lorsque m1 est 2 ou 3, alors les substituants R32 peuvent être identiques ou différents les uns des autres.

13. Procédé de préparation d'un composé de formule (VII) selon la revendication 12, dans lequel R1 est phényle ;
R2 est méthyle ;
R31 est tert-butoxy ;
m1 est 0.
